# EUROPEAN PATENT APPLICATION

(11) **EP 1 801 210 A1**
(43) Date of publication of application: **27.06.2007**
(21) Application number: 05781466.7
(22) Date of filing: 24.08.2005
(51) Int. Cl.: C12N 15/09

(54) **CONJUGATE OF PEO WITH DOUBLE-STRANDED NUCLEIC ACID**

(30) Priority: 01.09.2004 JP 2004254824
(71) Applicant: Japan Science and Technology Agency, Kawaguchi-shi, Saitama 332-0012 (JP)
(72) Inventor: NAGASAKI, Yukio, Moriya-shi, Ibaraki 302-0128 (JP); KATAOKA, Kazunori, Tokyo 165-0031 (JP); OISHI, Motoi, Tsukuba-shi, Ibaraki 305-0005 (JP)
(74) Representative: Hiebl, Inge Elisabeth
(86) International application number: PCT/JP2005/015831
(87) International publication number: WO 2006/025419

(57) **Abstract**

A polyion complex is provided, comprising a double stranded nucleic acid containing a poly(ethylene oxide)-modified oligo- or poly-nucleotide and a polycationic compound, which is an effective delivery means of a gene to a target cell.

## Description

### Technical Field

The present invention relates to a conjugate of double stranded nucleic acid modified with poly(ethylene oxide) and a polyion complex (also called PIC) of a polycationic compound comprising the conjugate and a polyamine. Such conjugate and PIC are used advantageously in biochemistry, chemotherapy, and in particular in methods for suppressing the expression of target genes in diverse animal cells.

### Background Art

Genes used in gene-specific therapy, or oligodeoxyribonucleotides that can be used with target mRNAs have been drawing attention as therapeutics. However, in the treatment, these genes and oligodeoxyribonucleotides (ODNs) do not always exert the anticipated effects due to various obstacles when delivering these to the target site, such as, for instance, non-specific interaction of ODNs and blood plasma proteins, low efficiency of incorporation into target cells accompanying the preferential incorporation of ODNs into liver and kidney, and low stability of genes and ODNs against degradation by nucleases.

As means to allow such obstacles to be eliminated successfully, block co-polymer micelle or polyion complex (PIC) micelle formed from an antisense DNA and a thiolated poly(ethylene glycol)-block-poly(L-lysine) has been proposed (for instance, refer to Patent Reference 1, Non-Patent Reference 1 or Non-Patent Reference 2, below). In addition, ODN-poly(ethylene glycol) conjugate formed through an acid-cleavable B-propionate bond, in addition, PIC micelle of the conjugate and linear poly(ethylene imine), are also strong candidates allowing the above obstacles to be eliminated (for instance, refer to Non-Patent Reference 3, below.). PIC micelles using the above block co-polymer are used mainly as carriers for stabilization or delivery of plasmid DNA or antisense DNA (single stranded), and in addition, mainly to improve stabilization of antisense DNA in Non-Patent Reference 3.

Meanwhile, double stranded RNA molecules, which are referred to as Small interference RNAs (siRNAs) considered not to provoke interferon induction of cells, efficiently trigger RNAi (sequence specific RNA interference or sequence specific gene expression inhibition) in cultured cells, and are drawing attention as a candidate substances for therapeutic drugs, from the fact that RNAi activity is significantly higher than antisense DNA (for instance, refer to Non-Patent Reference 4 and Non-Patent Reference 5, below.). In addition, it has been reported that RNAi was triggered also with double stranded DNA/RNA in which the sense strand of an siRNA was substituted with DNA (for instance, refer to Non-Patent Reference 6, below). However, degradation in blood, absorption to kidney and excretion have been reported to occur also for these double stranded nucleic acids, similarly to antisense DNAs (for instance, refer to Non-Patent Reference 7, below).
List of references quoted above:
Patent Reference 1: Japanese Patent Application Laid-open No. 2001-146556
Non-Patent Reference 1: Biomacromolecules 2001, 2, 491-497
Non-Patent Reference 2: J.Am.Chem. Soc.2004, 126, 2355-2361
Non-Patent Reference 3: Biomacromolecules 2003, 4, 1426-1432
Non-Patent Reference 4: Nature 2001, 411, 494-498
Non-Patent Reference 5: Biochemical and Biophysical Research Communications 2002, 296 (2002), 1000-1004
Non-Patent Reference 6: FEBS Letters 2002, 521, 195-199
Non-Patent Reference 7: Bioorganic and Medicinal Chemistry Letter 2004, 14, 1139-1143

### Disclosure of the Invention

An object of the present invention is to provide a method for improving in particular the stability of the above-mentioned double stranded nucleic acid in biological environment, and furthermore, enabling an efficient incorporation thereof into animal cells. When the extremity of either one of the oligo- or poly-nucleotides of a double stranded nucleic acid is modified by covalently bonding a segment having a poly(ethylene oxide) chain, and the conjugate of double stranded nucleic acid thus modified and a polycationic compound are mixed in a water based medium, they associate automatically to form polyion complex (PIC) micelles. It was discovered that with the PIC obtained in this way, the double stranded nucleic acid therein is stabilized in a biological environment, and furthermore, the incorporation efficiency into animal cells becomes higher.

Consequently, according to the present invention, a polyion complex comprising:
(A) a double stranded nucleic acid, which is a double stranded nucleic acid comprising two species of oligo- or poly-nucleotides having complementarity, wherein a segment having a poly(ethylene oxide) chain is covalently bonded to the extremity of at least one species of oligo- or poly-nucleotide (hereinafter also referred to as Constituent A);
   and
(B) a polycationic compound comprising a polyamine
(hereinafter also referred to as Constituent B)
is provided.

In addition, although the polyion complex (PIC) of the present invention can exist in micellar form in a water based medium, with a PIC formed using a compound having at least one mercapto group (-SH) on the side chain as polycationic compound forming the PIC, PIC micelles cross-linked with at least one disulphide bond (-SS-) between these compounds can be provided. Such PIC micelles are provided in an improved structural stability mode.

The PIC of the present invention has no limitation on the content ratio of Constituent A and Constituent B as long as it can form a micelle in a water based medium (for instance, physiological saline and phosphate-buffered physiological saline (PBS), PIC for which the ratio of anion derived from the phosphates in the double stranded nucleic acid of Constituent A and cation derived from the amine residues in the polycationic compound of Constituent B is 0.5/1 to 1/10 is provided as a preferred mode. If the entirety of a PIC micelle is slightly positively charged, the efficiency of incorporation of the micelle into animal cells often becomes high.

The double stranded nucleic acid is chosen from, respectively, a hybrid of oligo- or poly-ribonucleotide and oligo- or poly-ribonucleotide (RNA/RNA), a hybrid of oligo- or poly-deoxyribonucleotide and oligo- or poly-ribonucleotide (DNA/RNA), as well as a hybrid of oligo- or poly-deoxyribonucleotide and oligo- or poly-deoxyribonucleotide (DNA/DNA), of two complementary species.

The terms nucleic acid, nucleotide, ribonucleotide, deoxyribonucleotide, RNA and DNA used in relation to the present invention have the meanings generally used in the relevant art. In the present specification, the term oligo- or poly-ribonucleotide is used interchangeably with RNA, and the term oligo- or poly-deoxyribonucleotide is used interchangeably with DNA.

The compound of Constituent A in which a segment having a poly(ethylene oxide) chain is covalently bonded to the extremity of an oligo- or poly-nucleotide, can be represented more concretely with the following General Formula (1).

NT-L₁-L₂-PEO (1)

wherein NT represents an oligo- or poly-nucleotide residue bonded to L₁-L₂-PEO via a phosphoester bond at the 3' or 5' end of the ribose,
L₁ represents an alkylene group having a total of 3 to 30 atoms, which may be interrupted by an oxygen atom or a sulphur atom at one or two or more positions,
L₂ represents a linking group having a bond that can be cleaved under physiological conditions, and
PEO represents a poly(ethylene oxide) group for holding a hydrogen atom, an alkyl group, an aralkyl group, a functional group or a ligand residue, via a linking group depending on circumstances, at the terminal bond of L₂.

As Constituent A in a more concrete mode, the compound in which -PEO in the above General Formula (1) is represented by Formula (2):

-(CH₂CH₂O)ₙ-L₃-X (2)

where L₃ represents a single bond, a carbonyl or the same linking group as defined for L₁,
X is chosen from the group comprising C₁-C₆ alkyl, hydroxy-C₁-C₆ alkyl, carboxy-C₁-C₆ alkyl, acetalized or ketalized formyl-C₁-C₆ alkyl, amino-C₁-C₆ alkyl, maleimide-C₁-C₆ alkyl, carbonyl iminophenyl, allyl and portions comprising a ligand bonded through these functional groups, and
n represents an integer from 5 to 500, can be cited.

Meanwhile, although there is no limitation on the polycationic compound comprising the polyamine of Constituent B, it is selected from the group consisting of polylysine in which one or a plurality of SH groups have been introduced, polylysine, poly ethylene imine, poly dimethyl aminoethyl methacrylate, oligo-arginine, tat and KALA. Regarding the molecular weights of the polycationic compounds comprising these polyamines, as long as they associate with Constituent A to form PIC micelles, they can be used without limitation. However, if polylysine is taken as an example, they can be generally 500 to 100,000, preferably 1,000 to 5,0000, and more preferably 5,000 to 25,000. Regarding other polycations comprising other polyamines, the preferred molecular weight can be determined with the above example of polylysine as reference, by carrying out small experiments, as necessary. As polylysine in which one or a plurality of SH groups have been introduced on the side chain, those selected so that there is one SH group every 10 lysine units, preferably 5, can be cited.

In addition, a nucleic acid conjugate is also provided as the present invention in another mode, in which an oligo- or poly-nucleotide complementary to the NT portion of the compound represented by the General Formula (1):

NT-L₁-L₂-PEO (1)

(wherein NT represents an oligo- or poly-nucleotide residue bonded to L₁-L₂-PEO via a phosphoester bond at the 3' or 5' end of the ribose,
L₁ represents an alkylene group having a total of 3 to 30 atoms, which may be interrupted by an oxygen atom or a sulphur atom at one or two or more positions,
L₂ represents a linking group having a bond that can be cleaved under physiological conditions, and
PEO represents a poly(ethylene oxide) group for holding a hydrogen atom, an alkyl group, an aralkyl group, a functional group or a ligand residue, via a linking group depending on circumstances, at the terminal bond of L₂.)
hybridizes to form a double stranded nucleic acid portion is also provided.

As conjugates of the preferred mode, those in which -PEO in the above formula is represented by Formula (2)

-(CH₂CH₂O)n-L₃-X (2)

described for the above PIC can be cited.

### Brief Description of the Drawings

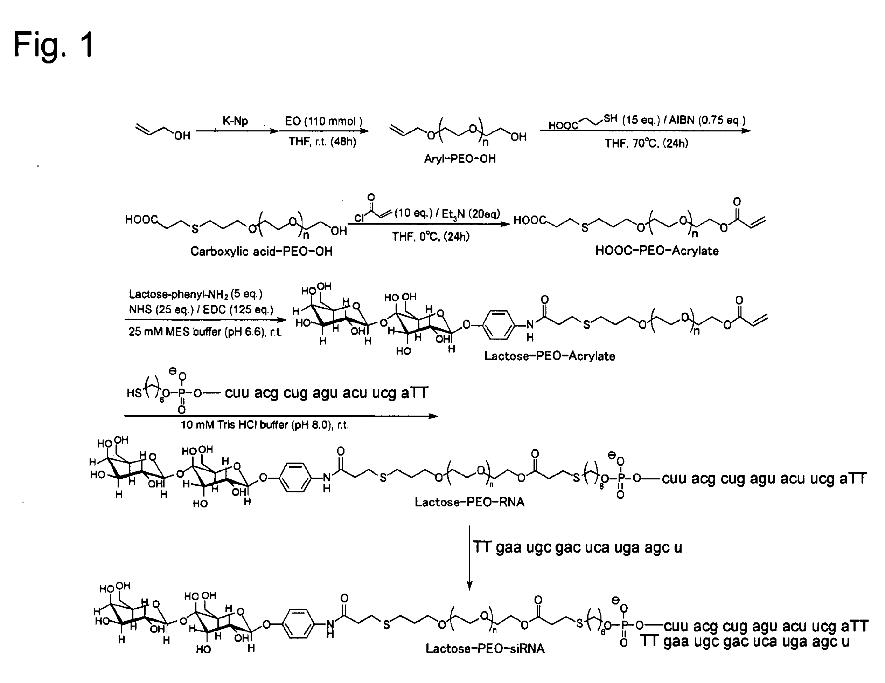

Fig. 1 is a synthesis scheme for lactose-PEG-siRNA conjugate.
Fig. 2 is the measurement result for lactose-PEG-acrylate by -NMR.
Fig. 3 is a photograph replacing a Fig. representing the result of 12% acrylamide gel electrophoresis of lactose-PEG-RNA conjugate, lactose-PEG-siRNA conjugate, and PIC micelle and S-S cross-linked PIC micelle.
Fig. 4 is the result of RNAi effects of lactose-PEG-DNA/RNA conjugate, lactose-PEG-siRNA conjugate, PIC micelle and S-S cross-linked PIC micelle, at 100nM.

### Detailed description of the Invention

The term "oligomer (or oligo) or poly" in oligo- or poly-nucleotide is used with the intent to include everything having a chain length to form a PIC and form a PIC micelle in a water based medium. It is therefore not limited, and concretely, [the number of] nucleotide units is 10 to 5000, preferably 18 to 50, particularly preferably 21 to 22 so as to approach the [number of] nucleotide units of siRNA, and, such morphology in which one to two nucleotides are overhanging at the 3' end and 5' end in a double strand formed by these two species of strands is preferred.

In the present invention, "having complementarity" does not necessarily mean that the entirety of each nucleotide pairing is strictly complementary to each other over the entirety of a double strand, and concretely, representing with base pairs, does not go so far as to require the entirety of each nucleotide pair to be formed by any from adenine (A) and uracil (U) or guanine (G) and cytosine (C) or adenine (A) and thymine (T) and guanine (G) and cytosine (C), and is used with the concept of also including those where double stranded nucleic acids hybridize under highly stringent conditions.

Regarding the segment having a poly(ethylene oxide) chain covalently bonding to the extremity of such oligo- or poly-nucleotide, the portion represented by -L₁-L₂-PEO in General Formula (1) above is relevant as a concretely one. Herein, L₁, L₂ and PEO are as already defined for General Formula (1), in addition, the preferred mode for -PEO is the one represented by Formula (2). Regarding "alkylene group having a total of 3 to 30 atoms, which may be interrupted by an oxygen atom or a sulphur atom at one or two or more positions" in these definitions, for instance, -CH₂CH₂CH₂-, -CH₂CH₂-O-CH₂CH₂-, -CH₂CH₂-(O-CH₂CH₂)₂-, -CH₂CH₂-S-CH₂CH₂-, -CH₂CH₂-S-(CH₂)₆-, and the like, can be cited. In addition, in "linking group having a bond that can be cleaved under physiological conditions" the bond is, for instance, any ester bond that can be cleaved at a low pH (6.0 to 5.0) in an endosome or a disulphide bond that can be cleaved under reducing conditions or in the presence of a reducing substance can be cited. A linking group having such a bond may have the bond at any position within the linking group, and, for instance, -OCH₂CH₂OCO-CH₂-, -OCH₂CH₂SSCH₂-, -OCH₂CH₂CH₂OCO-CH₂-, -SCH₂CH₂COO-CH₂-, -OCH₂CH₂NHCH₂COO-CH₂-, and the like can be cited.

The alkyl or alkyl portion referred to in C₁-C₆ alkyl and hydroxy-C₁-C₆ alkyl in the definition when -PEO is represented by Formula (2): -(CH₂CH₂O)ₙ-L₃-X mean methyl, ethyl, propyl, isopropyl, n-hexyl, and the like, and portions derived therefrom. L₃ includes linking groups defined for L₁ above, and in addition to these, can be -O-Ph-NHCO-(Ph represents phenyl), -OCH₂CH₂NHCO-, -NHCOCH₂CH₂-, and the like. In X, "acetalized or ketalized formyl" referred to mean a protected form of formyl or aldehyde, in addition, "carboxy" and "amino" may be protected by a protecting group such as those in common use in peptide synthesis, and the like. In addition, as far as the ligand bonded via these functional groups (forming an amide, an ester or an ether linkage), it can be a sugar or a peptide that can bind to a given cell surface, furthermore, it can be an antibody, an antigen, a hapten, a hormone, or the like.

PIC or PIC micelle specified above can be formed by merely mixing Constituent A and Constituent B in a water based medium. In addition, the conjugate of the present invention, which may also be Constituent A, can be prepared by, for instance, the methods described in Non-Patent Reference 3, in which some of the present inventors are included in the authors, by preparing a single stranded nucleic acid modified with a poly(ethylene oxide) chain-containing segment, and then hybridizing a complementarity strand to the nucleic acid portion.

Meanwhile, among the poly-cations of Constituent B, in order to introduce SH group on side chains of polyamine, can be performed by treating polyamine according to the methods described in Non-Patent References 1 and 2, in which, again, some of the present inventors are included in the authors.

The oligo- or poly-nucleotide forming the double stranded nucleic acid can be any of the sense strand and antisense strand of any gene from an animal, and particularly from a mammal. Such genes can be those that are already the subjects of antisense DNAs that have been provided with the purpose of treating various diseases. As such genes, genes for PKCα related to non-parvicellular lung cancer and the like, BCL-2 related to malignant melanoma and the like, ICAM-1 related to Crohn's disease, HCV related to hepatitis C, TNFα related to rheumatoid arthritis and psoriasis, adenosine A1 receptor related to asthma, c-raf kinase related to ovarian cancer and the like, H-ras related to pancreatic cancer and the like, c-myc related to coronary artery disease cancer, PKA RIα related to large bowel cancer, HIV related to acquired immunodeficiency syndrome, DNA methyl-transferase related to solid cancer, VEGF receptor related to cancer, ribonucleotide reductase related to kidney cancer, CMV IE2 related to CMV retinitis, MMP-9 related to prostate gland cancer, TGF beta 2 related to malignant glioma, CD49d related to multiple sclerosis, PTP-1B related to diabetes, c-myb related to cancer, EGFR related to breast cancer and the like, mdrl, autotaxin and GLUT-1 related to cancer, can be cited, without limitation.

For instance, in the PIC of the present invention, if one of the double stranded nucleic acids is a portion of the sense strand or the antisense strand of the above-mentioned gene, the expression of the gene can be inhibited effectively using the PIC of the present invention. Therefore, the present invention is useful in the treatment of disease associated with the expression of the above-mentioned gene.

Hereinafter, the present invention will be further illustrated.

### Example 1: Preparation of allyl-PEO-OH

Under argon, in a recovery flask, at room temperature, 1.0mmol (0.07ml) of allyl alcohol starter was added to 25ml of tetrahydrofuran (THF) solvent with a micro syringe, and 1.0mmol K-naphthalene (0.325mol/l-THF solution, 3.1ml) was added to perform metallization for 10 minutes. Then, 110mmol (5.5ml) of ethylene oxide (EO) was added, and stirring was performed for two days under cooling water to carry out anionic ring-opening polymerization. Purification was carried out by diethyl ether precipitation (21), aspiration filtration, benzene freeze-drying. The yield for this product was 4.4g (98%).

### Example 2: Preparation of carboxylic acid-PEO-OH

In 30ml of THF, 1.0mmol of allyl-PEO-OH (4.3g, Mₙ=4340), 15mmol of 3-mercapto propanoic acid (1.6g, 15-fold molar amount) and 0.75mol of azo bis isobutylonitrile (AIBN) (0.12g, 0.75-fold molar amount) were dissolved, and freezing-degassing was performed three times. Then, the reaction was carried out under argon at 70°C for two days. Thereafter, purification was carried out by 2-propanol precipitation (21), centrifugal separation (5000xg, 45 minutes), dialysis against pure water (cutoff molecular weight: 3500; water exchanged after 1, 2, 4, 6, 8 and 12 hours) and water freeze-drying. The yield for this product was 3.6g (82%).

### Example 3: Preparation of carboxylic acid-PEO-acrylate

Under argon, in a recovery flask, at room temperature, 4.5mmol of acryloyl chloride (0.36ml,10-fold molar amount) was dissolved in 5ml of THF. Then, to this solution was added drop wise a solution of 0.45mmo1 of carboxylic acid-PEO-OH (2.0g, Mₙ=4380) and 9.0mmol of triethylamine(1.3ml, 20-fold molar amount) dissolved in 15ml of THF, at 0°C over one hour. Thereafter, the reaction was carried out in the dark at 0°C for one day, then, purification was carried out by 2-propanol precipitation (11), centrifugal separation (5000xg, 45 minutes), dialysis against pure water (cutoff molecular weight: 3500; water exchanged after 1, 2, 4, 6, 8 and 12 hours) and water freeze-drying. The yield for this product was 1.6g (72%).

### Example 4: Preparation of lactose-PEO-acrylate

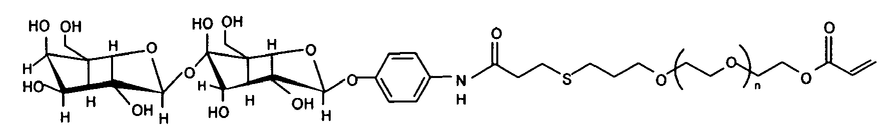

In a recovery flask, at room temperature, 22µmol of carboxylic acid-PEO-acrylate (0.1g, Mₙ=4450), 111µmol of 4-amino phenyl B-D-lactopyranoside (48mg, 5-fold molar amount), 2.8mmol of 1-(3-dimethylamino propyl)-3-ethyl carbodiimide hydrochloride (EDC) (0.53g, 125-fold molar amount) and 0.55mmol of N-hydroxy succinimide (NHS) (64mg, 25-fold molar amount) were dissolved in 5ml of 25mM 4-morpholino ethane sulfuric acid buffer solution (pH6.6) solvent, and the reaction was carried out at room temperature for one day. Thereafter, purification was carried out by 2-propanol precipitation (200ml), centrifugal separation (5000×g, 45 minutes), dialysis against pure water (cutoff molecular weight: 3500; water exchanged after 1, 2, 4, 6, 8 and 12 hours), water freeze-drying. The yield for this product was 69mg (67%).

By gel permeation chromatography measurement, the obtained polymer had a single-peak, and the number average molecular weight thereof was 4630, approximately corresponding to the theoretical molecular weight of 5490.

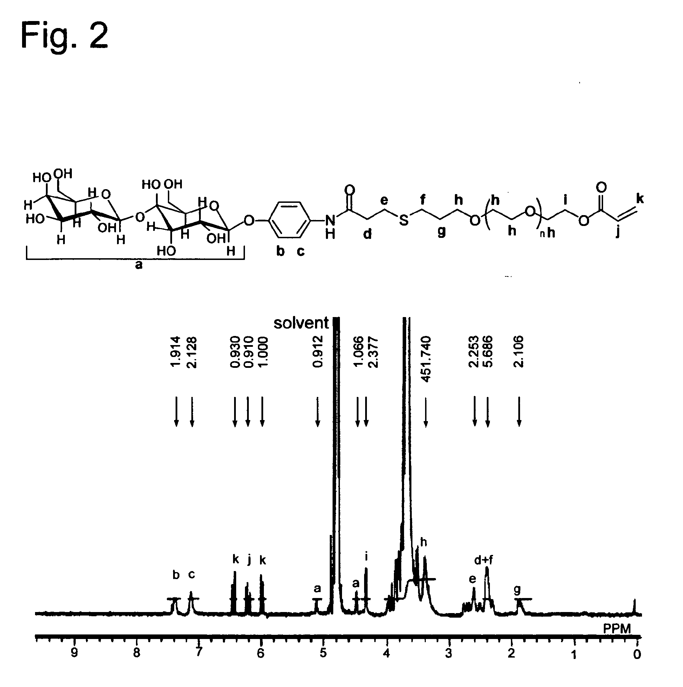

In addition, from the ¹H-NMR (proton nuclear magnetic resonance) spectrum in deuterium oxide (D₂O) of the obtained polymer, the average molecular weight of this polymer was calculated to be 5530. In addition this polymer was determined to be a heterotelechelic polyethylene oxide having an ethylene oxide backbone as main chain, a lactose group at the α-extremity, and an acrylate group at the ω-extremity (refer to Fig. 2).

### Example 5: Preparation of lactose-PEO-siRNA conjugate

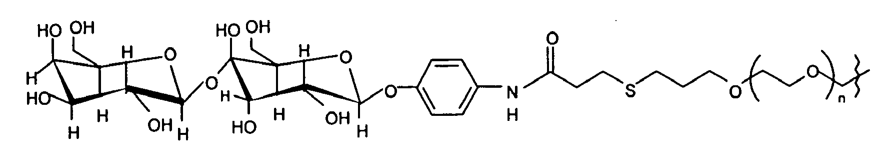

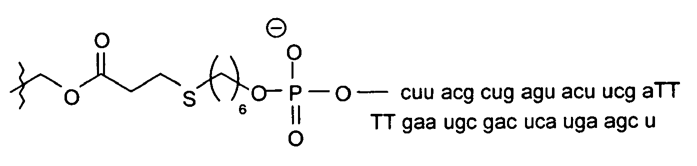

Into a test tube, 30nmol of 5'-end SH-modified sense strand RNA (193µg, purchased from Greiner Japan) and 0.3µmol of lactose-PEO-acrylate (1.6mg, 10-fold molar amount) were added, and argon exchange was carried out. Then, 300µl of 10nM tris-hydrochloric acid buffer solution (pH8) and 60µl of a 1mM triphenylphosphin-N,N-dimethyl formamide (DMF) solution (2-fold molar amount) were added with a micro syringe, and a Michael addition reaction was carried out at room temperature for two days. In addition, consumption of RNA and generation of RNA-PEO conjugate were checked by anion exchange chromatography. Thereafter, purification was carried out by fractionation by anion exchange column chromatography (Mono Q HR 10/10), and dialysis against pure water (cutoff molecular weight: 3500; water exchanged after 1, 2, 4, 6, 8 and 12 hours) for one day. The yield of this product by UV measurement was 89%. In addition, 27nmol of the obtained RNA-PEO conjugate and 27nmol of antisense strand RNA (purchased from Greiner Japan) were dissolved in 270µl of 10mM phosphate-buffered solution (pH7.4, annealing buffer), and annealed at 95°C for 3 minutes, to obtain lactose-PEO-siRNA conjugate quantitatively.

### Example 6: Preparation of SH-modified polylysine.

In 4ml of pure water, were dissolved 5.1µmol (42mg) of L-polysine hydrobromide salt having an average degree of polymerization of 40, 51µmol of Traut's reagent (7.0mg; 0.25-fold molar amount with respect to the amino groups of polylysine) and 4.5mmol (0.65ml) of triethylamine, and the reaction was carried out at for two days. Then, purification was carried out by dialysis against pure water (cutoff molecular weight: 3500; water exchanged after 1, 2, 4, 6, 8 and 12 hours), and water freeze-drying. The yield for this product was 40mg (82%).

In addition, from the ¹H-NMR (proton nuclear magnetic resonance) spectrum in deuterium oxide (D₂O) of the obtained polymer, the number of SH group (Traut's reagent) modifications on this polymer was determined to be 9 SH groups introduced per average degree of polymerization of 40.

### Example 7: Preparation of polyion complex (PIC) micelle comprising lactose-PEO-siRNA conjugate and polylysine

After dissolving 10nmol of lactose-PEO-siRNA conjugate and 12nmol of polylysine (average polymerization degree=40) respectively in 10mM tris-hydrochloric acid buffer solutions (pH7.4), dusts were removed by filtering with a 0.1µm filter. Then, these solutions were mixed so as to obtain a ratio of the positive charge of polylysine and the negative charge of lactose-PEO-siRNA conjugate of 1 (N/P=1). In addition, 10mM tris-hydrochloric acid buffer solution (pH7.4, 0.3M NaCl) was added in the same amount as the mixture, and left to stand for 12 hours to form PIC micelles.

### Example 8: preparation of S-S cross-linked polyion complex (PIC) micelle comprising lactose-PEO-siRNA conjugate and SH-modified polylysine.

After dissolving 10nmol of lactose-PEO-siRNA conjugate and 12nmol of SH-modified polylysine (average degree of polymerization=40) respectively in 10mM tris-hydrochloric acid buffer solution (pH7.4), dusts were removed by filtering with a 0.1µm filter. To this SH-modified polylysine solution, was added 12.5µl of 10mM tris-hydrochloric acid buffer solution (pH7.4) (3 times the molar equivalent of SH group) with 0.1µM dithiothreitol (DTT), and a reduction reaction was carried out at room temperature for 30 minutes. Next, after mixing so as to obtain a ratio of positive charge of polylysine and negative charge of lactose-PEO-SiRNA conjugate of 1 (N/P=1), dialysis (cut-off molecular weight: 3500) against 10mM tris-hydrochloric acid buffer solution (pH7.4) containing 0.5% dimethyl sulfoxide (DMSO) was carried out for two days. In addition, dialysis (cut-off molecular weight: 3500) against 10mM tris-hydrochloric acid buffer solution (pH7.4) containing 0.15M NaCl was carried out for one day to form an S-S cross-linked PIC micelle.

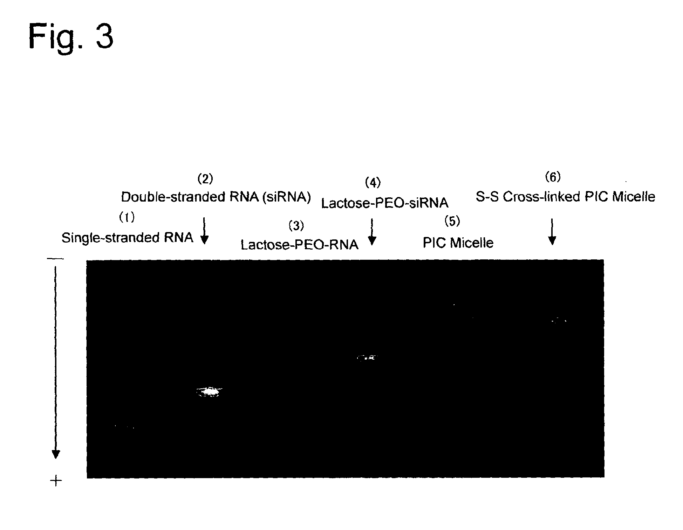

Verification of the preparation of lactose-PEO-RNA conjugate, lactose-PEO-siRNA conjugate, PIC micelle and S-S cross-linked PIC micelle was performed by 12% acrylamide electrophoresis (refer to Fig. 3). As a result, from the fact that the electrophoretic bands for the lactose-PEO-RNA conjugate and the lactose-PEO-siRNA conjugate were slower than the bands corresponding to the single-strand RNA and double-strand RNA (siRNA), the preparation of the conjugates was confirmed (comparison of lanes 1 and 3, and lanes 2 and 4). In addition, from the fact that the electrophoretic bands of the PIC micelle and S-S cross-linked PIC micelle were observed close to the starting point, the preparation of the PIC micelle and S-S cross-linked PIC micelle was confirmed (comparison of lanes 4 and 5, and lanes 4 and 6).

### Example 9: RNAi effects of lactose-PEO-siRNA conjugate and lactose-PEO-DNA/RNA conjugate PIC micelle

A 24-well polystyrene cell culture plate (Manufactured by Falcon) was inoculated with human hepatocellular cancer cell (Huh7 cell) at 5x10⁴ cell/well, culture was carried out for 24 hours, then, using the commercial transfection reagent Lipofect AMINE (1.22µL/well, manufactured by Invitrogen), cells were transfected with firefly luciferase plasmid DNA (pGL3, 0.084µg/well, manufactured by Promega) and Renilla luciferase plasmid DNA (pRL-TK, 0.75µg/well, manufactured by Promega) reporter gene. Then, prescribed amounts (converted in culture medium concentration: 100nM) of a solution of PIC micelle (prepared in Example 7), a solution of S-S cross-linked PIC micelle (prepared in Example 8) and a solution of conjugate alone, having sequences against the firefly luciferase gene were added, and left in contact with the cells for 24 hours. The culture medium was exchanged, then, culture was carried out for a further 24 hours, whereafter, the cells were recovered and the amount of expression of both reporter genes was measured with the Dual Luciferase Reporter Assay System (manufactured by Promega) to evaluate the antisense effect (n=3).

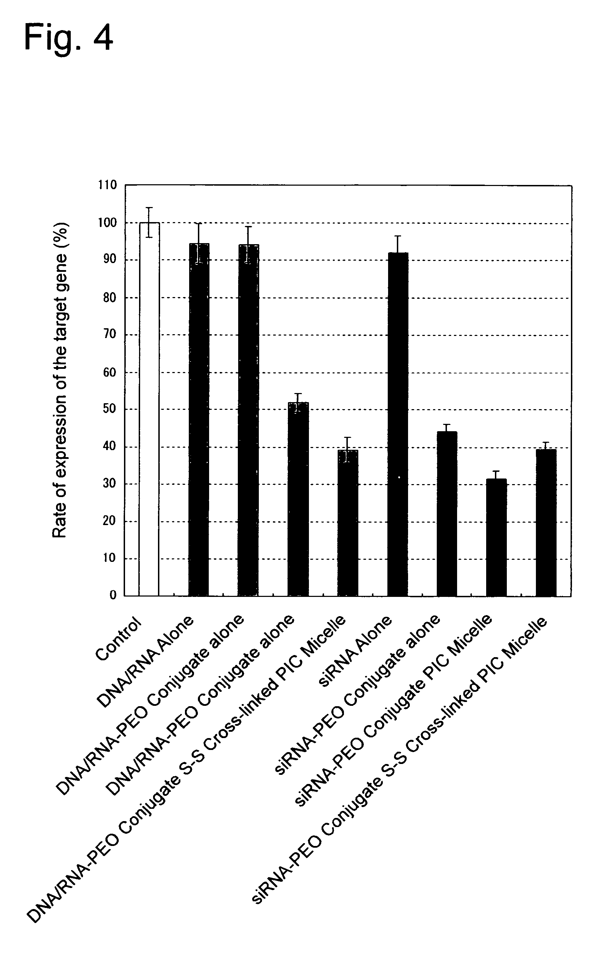

The results of the above are shown in Fig. 4. For DNA/RNA alone, siRNA alone and lactose-PEO-DNA/RNA conjugate alone, absolutely no demonstration of RNAi effect was observed. On the other hand, for the lactose-PEO-siRNA conjugate, demonstration of RNAi effect was observed.

For PIC micelle and S-S cross-linked PIC micelle, demonstration of RNAi effect was observed in all cases. In addition, a PIC micelle of a PEO conjugate having an siRNA was found to demonstrate a higher RNAi effect than a PEO conjugate PIC micelle having a DNA/RNA. In addition, S-S cross-linked PIC micelle of a PEO conjugate having an siRNA was also found to demonstrate similarly high RNAi effect.

### Industrial Applicability

PIC micelle of the present invention, allows an siRNA to be delivered efficiently to a target cell or tissue, can be used in the medical industry and the pharmaceutical industry.

## Claims

1. A polyion complex comprising:
(A) a double stranded nucleic acid, comprising two species of oligo- or poly-nucleotides having complementarity, in which a segment having a poly(ethylene oxide) chain is covalently bonded to the extremity of at least one species of oligo- or poly-nucleotide; and
(B) a polycationic compound comprising a polyamine.

2. The polyion complex according to Claim 1, wherein the polycationic compound comprising a polyamine is intermolecularly cross-linked with at least one disulphide bond.

3. The polyion complex according to Claim 1, wherein the ratio of anion deriving from the phosphates in the double stranded nucleic acid and cation deriving from the amine residues in the polycationic compound comprising a polyamine is 0.5/1 to 1/10.

4. The polyion complex according to Claim 1, wherein the polyion complex exists in the form of a micelle in a water based medium.

5. The polyion complex according to Claim 1, wherein the double stranded nucleic acid is a hybrid chosen from the group comprising RNA/RNA, DNA/RNA and DNA/DNA.

6. The polyion complex according to Claim 5, wherein the double stranded nucleic acid is an siRNA.

7. The polyion complex according to Claim 1, wherein the compound in which a segment having a poly(ethylene oxide) chain is covalently bonded to the extremity of an oligo- or poly-nucleotide is represented by the General Formula (1):
NT-LuL₂-PEO (1)
(wherein NT represents an oligo- or poly-nucleotide residue bonded to L₁-L₂-PEO via a phosphoester bond at the 3' or 5' end of the ribose,
L₁ represents an alkylene group having a total of 3 to 30 atoms, which may be interrupted by NH, an oxygen atom or a sulphur atom at one or two or more positions,
L₂ represents a linking group having a bond that can be cleaved under physiological conditions,
PEO represents a poly(ethylene oxide) group for holding a hydrogen atom, an alkyl group, an aralkyl group, a functional group or a ligand residue, via a linking group depending on circumstances, at the terminal bond of L₂.).

8. The polyion complex according to Claim 7, wherein -PEO is represented by the Formula (2)
-(CH₂CH₂O)ₙ-L₃-X (2)
(wherein L₃ represents a single bond, a carbonyl or the same linking group as defined for L₁,
X is chosen from the group comprising a hydrogen atom, C₁-C₆ alkyl, hydroxy-C₁-C₆ alkyl, carboxy-C₁-C₆ alkyl, acetalized or ketalized formyl-C₁-C₆ alkyl, amino-C₁-C₆ alkyl, maleimide-C₁-C₆ alkyl, carbonyl iminophenyl, allyl and portions comprising a ligand bonded through these functional groups, and
n represents an integer from 5 to 500).

9. The polyion complex according to Claim 7, wherein the oligo-or poly-nucleotide residue derives from an RNA or a DNA comprising 10 to 50 nucleotides.

10. The polyion complex according to Claim 1, wherein the polycationic compound comprising a polyamine is chosen from the group comprising polylysine in which one or a plurality of SH groups have been introduced in the side chain, polylysine, poly ethylene imine, poly dimethyl aminoethyl methacrylate, oligo arginine, tat and KALA.

11. A nucleic acid conjugate, wherein an oligo- or poly-nucleotide complementary to the NT portion of a compound represented by the General Formula (1)
NT-L₁-L₂-PEO (1)
(wherein NT represents an oligo- or poly-nucleotide residue bonded to L₁-L₂-PEO via a phosphoester bond at the 3' or 5' end of the ribose,
L₁ represents an alkylene group having a total of 3 to 30 atoms, which may be interrupted by an oxygen atom or a sulphur atom at one or two or more positions,
L₂ represents a linking group having a bond that can be cleaved under physiological conditions,
PEO represents a poly(ethylene oxide) group for holding a hydrogen atom, an alkyl group, an aralkyl group, a functional group or a ligand residue, via a linking group depending on circumstances, at the terminal bond of L₂.)
hybridizes to form a double stranded nucleic acid portion.

12. The nucleic acid conjugate according to Claim 11, wherein -PEO is represented by the Formula (2)
-(CH₂CH₂O)n-L₃-X (2)
(wherein
L₃ represents a single bond, a carbonyl or the same linking group as defined for L₁,
X is chosen from the group comprising a hydrogen atom, C₁-C₆ alkyl, hydroxy-C₁-C₆ alkyl, carboxy-C₁-C₆ alkyl, acetalized or ketalized formyl-C₁-C₆ alkyl, amino-C₁-C₆ alkyl, maleimide-C₁-C₆ alkyl and carbonyl iminophenyl group, as well as a portion comprising a ligand bonded through these functional groups, and
n represents an integer from 5 to 500).

13. The nucleic acid conjugate according to Claim 11, wherein NT is chosen from the group comprising nucleic acids comprising 15 to 30 ribonucleotides or deoxyribonucleotides, the oligonucleotide complementary to the NT portion being 15 to 30 ribonucleotides long, and L₂ being the linking group -O(CH₂)ₐ-OCO(CH₂)_{b}S(CH₂)_{c}- or -O(CH₂)ₐ-S-S(CH₂)c-, with a, b and c being mutually independent and representing an integer from 2 to 8.

14. The nucleic acid conjugate according to Claim 11, wherein the double stranded nucleic acid portion comprising NT and an oligonucleotide complementary thereto corresponds to an siRNA.
